(19) Europäisches Patentamt
European Patent Office
Office européen des brevets

(11) Publication number: **0 199 302**
**A2**

(12) # EUROPEAN PATENT APPLICATION

(21) Application number: 86105372.6

(22) Date of filing: 18.04.86

(51) Int. Cl.[4]: A 61 K 37/02
A 61 K 37/43

(30) Priority: 19.04.85 US 725267

(43) Date of publication of application:
29.10.86 Bulletin 86/44

(84) Designated Contracting States:
AT BE CH DE FR GB IT LI LU NL SE

(71) Applicant: SYNTEX (U.S.A.) INC.
3401 Hillview Avenue
Palo Alto, California 94304(US)

(72) Inventor: Vickery, Brian H.
20279 Carol Lane
Saratoga California 95070(US)

(74) Representative: Barz, Peter, Dr. et al,
Patentanwälte Dipl.-Ing. G. Dannenberg Dr. P. Weinhold,
Dr. D. Gudel Dipl.-Ing. S. Schubert, Dr. P. Barz
Siegfriedstrasse 8
D-8000 München 40(DE)

(54) **Contraception in dogs with luteinizing hormone releasing hormone antagonists.**

(57) A LHRH antagonist is used for the manufacture of a contraceptive medicament for dogs.

EP 0 199 302 A2

# CONTRACEPTION IN DOGS WITH LUTEINIZING HORMONE RELEASING HORMONE ANTAGONISTS

This invention relates to contraception in dogs using luteinizing hormone releasing hormone (LHRH) antagonists.

The most common and popular method of preventing pregnancy in the bitch is ovariohysterectomy. This has the disadvantage of being irreversible and is unacceptable in breeding stock. Prostaglandins will terminate pregnancy, if administered in mid-gestation or later, however this action is accompanied by intolerable side effects including emesis, diarrhea and changes in thermoregulatory and cardiovascular parameters (Vickery & McRae Biol. Reprod. 22:438-442, 1980). The cardiovascular side effects in particular result in very low therapeutic ratio. Estrogens have also been used very shortly after mating as an interceptive ("mis-mating"). Again, concerns over induction of nymphomania, and problems of carcinogenesis and cardiovascular complication, particularly thrombogenesis, leave these steroids contraindicated for this use.

Progestational steroids were useful as long term estrus suppressants (Harris and Wotchuk Am. J. Vet. Res. 24:1003-1006, 1963). Unfortunately the noted induction of uterine disorders including pyometritis and endometritis (Anderson, Gillmore and Schnelle J. Am. Vet. Med Assoc. 146:1311-1316, 1965) and increased incidence of benign mammary tumors associated with long term treatment has resulted in their present use merely as short term postponement of estrus (Burke and Reynolds J. Am. Vet. Med Assoc. 167:285-287, 1975). An androgenic steroid, Mibolerone •, is available commercially for long term estrus suppression in bitches but again the inconvenience of daily administration, high interbreed differences in dosage requirements and the masculinizing effects on hair quality and secondary sexual features have combined to impede wide scale usage of the agent (Sokolowski and Geng Am. J. Vet. Res. 38:1371-1376, 1977).

Thus there is a need for a well tolerated, reversible, non-steroidal method of contraception in female dogs. Such a method would ideally be able to both terminate and suppress estrus and therefore breeding as well as terminate an ongoing pregnancy. As in many cases the exact date of breeding is not known, in the case of unwanted pregnancy, the ideal agent would be effective in terminating gestation when administered at any time during pregnancy.

Luteinizing hormone (LH) and follicle stimulating hormone (FSH) are the two gonadotropic hormones which regulate gametogenesis and gonadal steroidogenesis and are therefore responsible for reproductive cyclicity and for all or part of the course of gestation in mammals. Luteinizing hormone releasing hormone (various synonyms include LH-RH; GnRH; LH/FSH-RH, but LHRH will be used herein) controls the synthesis and release of LH and FSH;

therefore LHRH antagonists can interfere with the reproductive cycle.

LHRH occurs naturally as a decapeptide comprised of naturally occurring amino acids (which have the L-configuration except for the achiral amino acid glycine). Its sequence is as follows:

(pyro) Glu-His-Trp-Ser-Tyr-Gly-Leu-Arg-Pro-Gly-$NH_2$
1 2 3 4 5 6 7 8 9 10

Analogs have been prepared which are competitive antagonists to LHRH; all of which require deletion or replacement of the histidine residue at position 2 (Vale, W., et al, Science, 176: 933 ,1972).

It has also been shown that adding a modification at the 6 position enhances the antagonist activity of the 2-modified analogs (Beattie, C. W., et al, J. Med. Chem., 18: 1247, 1975; Rivier, J., et al, Peptides 1976 p. 427, Editions de l'Universite de Bruxelles, Belgium, 1976).

Against the background of these two major alterations, which result in a potent series of LHRH antagonists; additional increments in antagonist activity may be had by modifying positions 1, 3 and/or 10 in the already 2, 6 modified peptide. See for example, Coy, D. H., et al Peptides 1976, p. 462, Editions de l'Universite de Bruxelles, Belgium, 1976; Rivier, J. E., et al, Life Sci. 23: 869, 1978. It has also been shown that N-acylation of the amino acid at position 1 is helpful. See for example, Coy, D. H., et al, Peptides. - Structure and Biological Function p. 775, Pierce Chemical Co., 1979. In addition, hydrophobic substitutions at position 7 have been found to be useful. See for example, Folker, K. et al, Biochem. Biophys. Res. Commun., 123, 1221-1226,1984.

Additionally, (N-Ac-D-p-Cl-$Phe^{1}$, D-p-Cl-$Phe^{2}$, D-$Trp^{3}$, D-$Arg^{6}$, D-$Ala^{10}$)LHRH has been reported

by D.H. Coy et al., Endocrinology, 110, 1445 (1982). A series of highly potent LHRH antagonists were disclosed in European Patent Application No. 83303343.4, including [N-Ac-D-Nal(2)$^{1}$, D-p-Cl-Phe$^{2}$, D-Trp$^{3}$, D-Deh$^{6}$, D-Ala$^{10}$]LHRH. These compounds were disclosed as having a range of effects and utilities in animals, including contraception, ovulation inhibition, estrus suppression and growth promotion, among various other uses.

Although one might expect LHRH antagonists to work equally well in regulating the reproductive cycle of all mammals, the developing feto-placental unit of certain mammalian species is known to exercise autonomous control of the pregnancy by independently eliminating the requirement of LH and FSH. Unexpectedly, studies with LHRH antagonists have now shown that the antagonists work particularly well to terminate pregnancy in dogs, but are ineffective in terminating pregnancy in cats.

This invention provides a process for contraception in female dogs comprising administrating an LHRH antagonist either during estrus or during pregnancy for a period of time sufficient to terminate either estrus or pregnancy.

ABBREVIATIONS AND DEFINITIONS

As set forth above, and for convenience in describing this invention, the conventional abbreviations for the various common amino acids are used as generally accepted in the peptide art as recommended by the IUPAC-IUB Commission on Biochemical Nomenclature, Biochemistry, 11, 1726 (1972). These, except for Gly

which represents the achiral amino acid glycine, represent the naturally occurring L-amino acids. All peptide sequences mentioned herein are written according to the generally accepted convention whereby the N-terminal amino acid is on the left and the C-terminal amino acid is on the right.

Certain other abbreviations will be useful in describing the invention. The present invention employs replacements by amino acids which do not occur in nature. Particularly commonly employed among these are the following:

| Amino acid residue | Abbreviation |
|---|---|
| 3-(2-naphthyl)-D-alanyl | D-Nal(2) |
| 3-(p-fluorophenyl)-D-alanyl | D-p-F-Phe |
| 3-(p-chlorophenyl)-D-alanyl | D-p-Cl-Phe |
| 3-(p-bromophenyl)-D-alanyl | D-p-Br-Phe |
| 3-(4-(trifluoromethylphenyl)-D-alanyl | D-Ptf |
| $N^G,N^{G'}$-diethyl-D-homoarginine | D-Deh |
| $N^G,N^{G'}$-bis(2,2,2-trifluoroethyl)-D-homoarginine | D-FDeh |
| 3-(3-pyridyl)-D-alanyl | D-Pal(3) |

Those amino acids that are the D- configuration of naturally occurring amino acids will be so noted.

As a further convenience, since the amino acid sequence of LHRH has been shown to be

(pyro)Glu-His-Trp-Ser-Tyr-Gly-Leu-Arg-Pro-Gly-$NH_2$,
1 2 3 4 5 6 7 8 9 10

nona- and decapeptides in which the amino acid residues at particular places in the sequence have been replaced by other amino acid residues or other moieties are abbreviated by showing the nature of the substitution, superscribed by the location, followed by LHRH as the parent.

Thus, for example, the sequence,

(pyro)Glu-D-p-F-Phe-Trp-Ser-Tyr-D-Deh-Leu-Arg-Pro-Gly$NH_2$
1 2 3 4 5 6 7 8 9 10

in which the Gly at position 6 has been replaced by D-Deh and the His at position 2 has been replaced by D-p-F-Phe, is represented [D-p-F-$Phe^2$, D-$Deh^6$]LHRH; and the sequence

N-Ac-D-Nal(2)-D-pCl-Phe-D-Trp-Ser-Tyr-D-Deh-
1 2 3 4 5 6

-Leu-Arg-Pro-D-Ala
7 8 9 10

is represented: [N-Ac-D-$Nal(2)^1$, D-p-Cl-$Phe^2$, D-$Trp^3$, D-$Deh^6$, D-$Ala^{10}$]LHRH.

As used herein, the term "pharmaceutically acceptable salts" refers to salts that retain the desired biological activity of the parent compound and do not impart any undesired toxicological effects. Examples of such salts are:

(a) acid addition salts formed with inorganic acids, for example hydrochloric acid, hydrobromic acid, sulfuric acid, phosphoric acid, nitric acid and the like; and salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, naphthalenesulfonic acids, naphthalenedisulfonic acids, polygalacturonic acid;

(b) salts with polyvalent metal cations such as zinc, calcium, bismuth, barium, magnesium, aluminum,

copper, cobalt, nickel, cadmium, and the like; or with an organic cation formed from N,N'-dibenzylethylene-diamine or ethylenediamine; or

(c) combinations, of (a) and (b), e.g., a zinc tannate salt and the like.

"Halo lower alkyl" refers to a lower alkyl radical substituted with halo groups, especially those having one, two or three halo groups on the ω-carbon. The halo group may be fluoro, chloro or bromo. This group is exemplified by trifluoromethyl, 2,2,2-trifluoroethyl, 3,3,3-trifluoropropyl, 2,2,2-trichloroethyl and the like.

DESCRIPTION OF THE ANALOGS

Some preferred compounds of the present invention include the class of compounds defined by [N-Ac-D-Nal(2)$^1$, D-p-Halo-Phe$^2$, D-Trp$^3$, D-Deh$^6$, D-Ala$^{10}$]LHRH, wherein the D-Deh$^6$ substituent is optionally substituted at the alkyl position with a halogen, and are described in European Patent Application Nos. 83303343.4 and 85114386.7.

Specific particularly preferred compounds include:

[N-Ac-D-Nal(2)$^1$, D-p-Cl-Phe$^2$, D-Trp$^3$, D-Deh$^6$, D-Ala$^{10}$]LHRH;

[N-Ac-D-Nal(2)$^1$, D-p-F-Phe$^2$, D-Trp$^3$, D-Deh$^6$, D-Ala$^{10}$]LHRH;

[N-Ac-D-Nal(2)$^1$, D-p-Cl-Phe$^2$, D-Trp$^3$, D-FDeh$^6$, D-Ala$^{10}$]LHRH; and

[N-Ac-D-Nal(2)$^1$, D-p-Cl-Phe$^2$, D-Trp$^3$, D-Deh$^6$, aza-Gly$^{10}$]LHRH.

Other preferred compounds include:

[N-Ac-Δ$^3$-Pro$^1$, D-pF-Phe$^2$, D-Trp$^{3,6}$, NMe-Leu$^7$]LHRH;

[N-Ac-Δ$^3$-Pro$^1$, D-pF-Phe$^2$, D-Nal(2)$^{3,6}$]LHRH;

[N-Ac-Pro$^1$, D-pF-Phe$^2$, D-Nal(2)$^{3,6}$]LHRH;

[N-Ac-Ala$^{1}$, D-pF-Phe$^{2}$, D-Trp$^{3,6}$]LHRH;

[N-Ac-D-Trp$^{1}$, D-pCl-Phe$^{2}$, D-Trp$^{3}$, D-Phe$^{6}$, D-Ala$^{10}$]LHRH;

[N-Ac-D-Trp$^{1}$, D-pCl-Phe$^{2}$, D-Trp$^{3}$, D-Arg$^{6}$, D-Ala$^{10}$]LHRH;

[N-Ac-D-pCl-Phe$^{1}$, D-pCl-Phe$^{2}$, D-Trp$^{3}$, D-Arg$^{6}$, D-Ala$^{10}$]LHRH;

[N-Ac-D-Nal(2)$^{1}$, D-pF-Phe$^{2}$, D-Trp$^{3}$, D-Arg$^{6}$]LHRH;

[N-Ac-D-Nal(2)$^{1}$, D-pF-Phe$^{2}$, D-Trp$^{3}$, oCl-Phe$^{5}$, D-Arg$^{6}$]LHRH;

[N-Ac-D-pBr-Phe$^{1}$, D-pCl-Phe$^{2}$, D-Trp$^{3}$, D-Arg$^{6}$, D-Ala$^{10}$]LHRH;

[N-Ac-D-Nal(2)$^{1}$, D-pCl-Phe$^{2}$, D-Trp$^{3}$, D-Arg$^{6}$, D-Ala$^{10}$]LHRH;

[N-Ac-D-Nal(2)$^{1}$, D-pCl-Phe$^{2}$, D-Pal(3)$^{3}$, D-Arg$^{6}$, D-Ala$^{10}$]LHRH;

[N-Ac-D-Nal(2)$^{1}$, D-pCl-Phe$^{2}$, D-Pal(3)$^{3}$, D-Arg$^{6}$, D-Trp$^{7}$, D-Ala$^{10}$]LHRH;

[N-Ac-D-Nal(2)$^{1}$, D-pCl-Phe$^{2}$, D-Pal(3)$^{3}$, D-Arg$^{6}$, D-Ile$^{7}$, D-Ala$^{10}$]LHRH;

[N-Ac-D-Nal(2)$^{1}$, D-pCl-Phe$^{2}$, D-Pal(3)$^{3}$, L-Arg$^{5}$, D-Glu(p-MeO-Phe)$^{6}$, D-Ala$^{10}$]LHRH;

[N-Ac-D-Nal(2)$^{1}$, D-α-Me-pCl-Phe$^{2}$, D-Pal(3)$^{3}$, iso-propyl-Lys$^{5,8}$, D-Tyr$^{6}$, D-Ala$^{10}$]LHRH;

[N-Ac-D-Nal(2)$^{1}$, D-αMe-pCl-Phe$^{2}$, D-Trp$^{3}$, Arg$^{5}$, D-Tyr$^{6}$, D-Ala$^{10}$]LHRH; and

[N-Ac-D-Nal(2)$^{1}$, D-αMe-pCl-Phe$^{2}$, D-Pal(3)$^{3}$, D-Arg$^{6}$, N-iPrLys$^{8}$, D-Ala$^{10}$]LHRH.

USE OF THE COMPOUNDS

The LHRH antagonist compounds of this invention can be used to terminate pregnancy, to terminate heat or estrus, or to treat pyometritis and endometritis.

To terminate a pregnancy, the endogenous LHRH action of the pregnant bitch must be antagonized for several days, thereby suppressing the luteal function and gonadal steroid production. This may be achieved by either once daily administration or continuous infusion of a suitable amount of an LHRH antagonist for seven days during mid to late gestation, for example, of 50-350 µg/Kg/day. In these instances delivery of an abortus normally results in 4 to 8 days. In the case of treatment during the first half of pregnancy the products of conception are resorbed and delivery does not occur. Treatment during very early stages of gestation may require increased dosage and/or longer duration of treatment to be effective, for example, 250-350 µg/kg/day, for at least seven days. In further studies it has unexpectedly been found that a single administration of an amount of LHRH antagonist equivalent to 1 to 2 times the sum of the daily doses is equally effective in terminating pregnancy in bitches.

If the LHRH antagonist is administered late in pregnancy close to term, the pregnancy terminating activity may be used for inducing delivery. This may be particularly useful when a large litter is expected or other complications are present such that it would be advantageous to the bitch or offspring to induce delivery in the presence of qualified veterinary supervision.

In order to terminate estrus, daily administration for 7-14 days of a suitable amount of LHRH antagonist, preferably, 50-350 µg/kg/day (or alternatively a single injection of an amount equal to 1 to 2 times the sum of the daily doses, for example, a single injection of between 1500 µg/Kg to 4500 µg/Kg, preferably,

1500-2500 μg/Kg) is given at any time during an estrous episode, but preferably at the first signs of sanguineous vaginal discharge. Such treatment results in a rapid return to a diestrous state, as judged by disappearance of discharge, a regression of vaginal cytology, an unwillingness of the bitch to accept a stud male or to breed, and a disappearance of attractiveness of the bitch to the male. In addition, ovulation does not occur.

The method may also be used to treat pyometritis and endometritis. When used for this treatment, it may be used in conjunction with the conventional treatment with prostaglandins. In the treatment of this invention the bitch is treated with the LHRH antagonist followed at least two hours later by conventional prostaglandin used for treatment of pyometritis and endometritis. The dose of prostaglandin required is directly related to the endogenous levels of progesterone, and as the plasma progesterone levels are precipitously decreased by the LHRH antagonist, therefore the therapeutic dosage requirement of the prostaglandins is reduced. Reduction of dosage of the prostaglandins leads in turn to a reduced incidence of side effects.

Compositions for use in this invention contain as active ingredient a compound of the present invention which compositions comprise such a compound in admixture with a pharmaceutically acceptable, non-toxic carrier. Such compositions may be prepared for use for parenteral (including subcutaneous, intramuscular or intravenous) administration particularly in the form of liquid solutions or suspensions; for use in vaginal or rectal administration particularly in semisolid forms such as creams and suppositories; or for oral or buccal administration particularly in the form of tablets or capsules.

The compositions may conveniently be administered in unit dosage form and may be prepared by any of the methods well-known in the pharmaceutical art, for example as described in Remington's Pharmaceutical Sciences, Mack Publishing Company, Easton, PA., 1970. Formulations for parenteral administration may contain as common excipients sterile water or saline, polyalkylene glycols such as polyethylene glycol, oils of vegetable origin, hydrogenated naphthalenes and the like. Formulations for vaginal or rectal administration, e.g. suppositories, may contain as excipients, for example, polyalkyleneglycols, vaseline, cocoa butter, and the like. For buccal administration typical excipients include sugars, calcium stearate, magnesium stearate, pregelatinated starch, and the like.

A dosage form may contain a pharmaceutically acceptable non-toxic salt of the compound which has a low degree of solubility in body fluids, for example, (a) an acid addition salt with a polybasic acid such as phosphoric acid, sulfuric acid, citric acid, tartaric acid, tannic acid, pamoic acid, alginic acid, polyglutamic acid, naphthalene mono- or di-sulfonic acids, polygalacturonic acid, and the like; (b) a salt with a polyvalent metal cation such as zinc, calcium, bismuth, barium, magnesium, aluminum, copper, cobalt, nickel, cadmium and the like, or with an organic cation formed from e.g., N,N'-dibenzylethylenediamine or ethylenediamine; or (c) combinations of (a) and (b) e.g. a zinc tannate salt.

Additionally, the compositions generally contain from 0-50 mg/ml, preferably from 20 µg/ml - 10 mg/ml, of the LHRH antagonist compound.

## EXAMPLES

### EXAMPLE 1

A bitch was bled and the plasma progesterone measured in nanograms per milliliter versus the day of diestrus. The bitch was bled on day -8 and day -4. The plasma progesterone was 4 to 6 nanograms per milliliter on those two days. At day 1, the plasma progesterone was at 30 nanograms per milliliter and peaked at about 60 on day 9 slowly decreasing to about 30 on day 25. At day 25 the bitch started to receive daily subcutaneous injections of $[N\text{-}Ac\text{-}D\text{-}Nal(2)^1, D\text{-}p\text{-}Cl\text{-}Phe^2, D\text{-}Trp^3, D\text{-}Deh^6, D\text{-}Ala^{10}]$LHRH for 7 days. After the first injection the plasma progesterone level immediately dropped to 4 nanograms per milliliter. At the end of the course of treatment, the level of plasma progesterone was down below 1 nanogram per milliliter. A fetus was expelled the 5th day of treatment and a tissue mass was expelled on day 49, which was 24 days after the start of treatment.

EXAMPLE 2

Several pregnant beagle bitches were treated with one of two different LHRH antagonists by subcutaneous injection. The results are summarized in Table 1.

Table 1

EFFECT OF TIME OF DOSING WITH TWO DIFFERENT LHRH ANTAGONISTS ON PREGNANCY IN BITCHES

| DOGS # | TREATMENT ON DAYS OF PREGNANCY | COMMENT |
|---|---|---|
| [N-Ac-D-Nal(2)$^{1}$ ,D-pCl-Phe$^{2}$ ,D-Trp$^{3}$ , D-Deh$^{6}$, Ala$^{10}$]-LHRH | | |
| 1 | 36-42* | Aborted on days 39-41 of pregnancy |
| 2 | 32-38* | Aborted on day 35 of pregnancy |
| [N-Ac-D-Nal(2)$^{1}$, D-pCl-Phe$^{2}$, D-Pal(3)$^{3}$, D-Deh$^{6}$, D-Ala$^{10}$]LHRH | | |
| 3 | 29-35† | Resorbed, discharge day 35 |
| 4 | 28-34† | Resorbed, discharge day 34 |

* daily injection for seven days, 300 µg/kg/day

†continuous infusion for seven days, 300 µg/kg/day

EXAMPLE 3

A beagle bitch was bred and confirmed to be pregnant by manual palpation of the uterus during the fourth week of gestation. On the twenty-ninth day of pregnancy she was administered a single subcutaneous injection of an aqueous solution of 50 mg of [N-Ac-D-Nal(2)$^1$, D-p-Cl-Phe$^2$, D-Trp$^3$, D-Deh$^6$, D-Ala$^{10}$]LHRH. Immediately before injection the plasma progesterone levels was 19ng/ml and by 24 hours after injection this had declined to 3.3 ng/ml. Plasma levels of progesterone varied thereafter from day to day but were never above 6 ng/ml. Delivery of an abortus was noted on the 36th day of pregnancy.

EXAMPLE 4

A beagle bitch was identified with a slight sanguineous vaginal discharge (day 1 of the experiment). Microscopic evaluation of vaginal cytology confirmed the bitch to be in proestrus. On day 2 the vaginal cytology showed the bitch to be entering estrus. She was immediately started on a course of treatment of 300 µg/kg/day for 14 days of [N-Ac-D-Nal(2)$^1$, D-p-Cl-Phe$^2$, D-Trp$^3$, D-Deh$^6$,D-Ala$^{10}$]LHRH. The vaginal discharge diminished and was last noted on day 5. By day 6 the bitch was confirmed by vaginal cytology to have reentered diestrus. The bitch exhibited no sign of sexual receptivity and was not attractive to stud males.

## EXAMPLE 5

Female cats were bred and 15-18 days later were implanted subcutaneously with an Alzet• minipump filled with a solution of [N-Ac-D-Nal(2)$^{1}$, D-p-Cl-Phe$^{2}$, D-Trp$^{3}$, D-Deh$^{6}$,D-Ala$^{10}$]LHRH in propylene glycol at a concentration sufficient to deliver 300 µg/kg/day. The pumps were removed after seven days. No effect of treatment on circulating levels of progesterone could be discerned in daily plasma samples. All treated cats continued to normal uneventful term delivery.

TABLE

Effect of 300 µg/kg/day of [N-Ac-D-Nal(2)$^{1}$, D-p-Cl-Phe$^{2}$,D-Trp$^{3}$, D-Deh$^{6}$,D-Ala$^{10}$]LHRH for seven days on plasma levels of progesterone in pregnant cats.

| Days from Treatment | Plasma level of progesterone (ng/ml) Cat 1 | Cat 2 |
|---|---|---|
| -2 | 4.5 | 4.3 |
| -1 | 2.0 | 3.0 |
| 0 | 4.0 | 2.3 |
| 1 | 2.7 | 4.1 |
| 2 | 4.3 | 5.2 |
| 3 | 4.0 | 3.9 |
| 4 | 4.4 | 4.2 |
| 5 | 3.8 | 4.2 |
| 6 | 5.7 | 6.3 |
| 7 | 3.3 | 5.5 |
| 8 | 6.5 | 6.1 |
| 9 | 6.7 | 7.3 |
| 10 | 5.2 | 6.7 |

This test shows that no significant effects were obtained in female cats administered the LHRH-antagonist.

EXAMPLE 6

Typical pharmaceutical compositions contain as active ingredient an LHRH antagonist, for example N-Ac-D-Nal(2)-D-pCL-Phe-D-Trp-Ser-Tyr-D-Deh-Leu-Arg-Pro-D-Ala, by itself or as a pharmaceutically acceptable salt, e.g. the acetic acid addition salt, the zinc salt, the zinc tannate salt, etc.

The following is an example of a composition containing the acid addition salt:

| Subcutaneous Injectable Solution | |
|---|---|
| LHRH Antagonist | 10.0 mg |
| Benzyl Alcohol | 9.0 mg |
| Glacial Acetic Acid | 1.2 mg |
| Propylene Glycol | 200.0 mg |
| Mannitol | 35.0 mg |
| Sterile Water for injection | 1.0 ml |

Method of Manufacture

A. Preparation of Vehicle:

Benzyl alcohol is dissolved in approximately 50% of the water, at about 40°C., then propylene glycol and mannitol are each successively dissolved in this solution, maintaining at 40°C. The resulting solution is cooled to room temperature and acetic acid is added. The pH of the solution is adjusted to 5.0 ± 0.2 with 1.0 N NaOH, and brought to volume with sterile water.

B. Preparation of Active Composition:

The LHRH antagonist is dissolved in 90% of the prepared vehicle, and the pH is adjusted to 5.0 ± 0.2 with 1.0 N NaOH. The volume is brought up with the vehicle, the solution is mixed and filtered through a suitable filter. Then, 5 ml clear type-I multiple-dose vials are aseptically filled with 5.2 ml of the final solution, stoppered and sealed, and autoclaved for sterility.

Claims:

1. The use of a LHRH antagonist for the manufacture of a contraceptive medicament for female dogs.

2. The use of claim 1 wherein the LHRH antagonist is selected from
[N-Ac-D-Nal(2)$^{1}$, D-p-Halo-Phe$^{2}$, D-Trp$^{3}$, D-Deh$^{6}$ (optionally substituted at the alkyl position with a halogen), D-Ala$^{10}$]LHRH.

3. The use of claim 2 wherein the LHRH antagonist is selected from:

[N-Ac-D-Nal(2)$^{1}$, D-p-Cl-Phe$^{2}$, D-Trp$^{3}$, D-Deh$^{6}$, D-Ala$^{10}$]LHRH;

[N-Ac-D-Nal(2)$^{1}$, D-p-F-Phe$^{2}$, D-Trp$^{3}$, D-Deh$^{6}$, D-Ala$^{10}$]LHRH;

[N-Ac-D-Nal(2)$^{1}$, D-p-Cl-Phe$^{2}$, D-Trp$^{3}$, D-FDeh$^{6}$, D-Ala$^{10}$]LHRH; and

[N-Ac-D-Nal(2)$^{1}$, D-p-Cl-Phe$^{2}$, D-Trp$^{3}$, D-Deh$^{6}$, aza-Gly$^{10}$]LHRH.

4. The use of claim 1 wherein the LHRH antagonist is selected from:

[N-Ac-$\Delta^{3}$-Pro$^{1}$, D-pF-Phe$^{2}$, D-Trp$^{3,6}$, NMe-Leu$^{7}$]LHRH;

[N-Ac-$\Delta^{3}$-Pro$^{1}$, D-pF-Phe$^{2}$, D-Nal(2)$^{3,6}$]LHRH;

[N-Ac-Pro$^{1}$, D-pF-Phe$^{2}$, D-Nal(2)$^{3,6}$]LHRH;

[N-Ac-Ala$^{1}$, D-pF-Phe$^{2}$, D-Trp$^{3,6}$]LHRH;

[N-Ac-D-Trp$^{1}$, D-pCl-Phe$^{2}$, D-Trp$^{3}$, D-Phe$^{6}$, D-Ala$^{10}$]LHRH;

[N-Ac-D-Trp$^{1}$, D-pCl-Phe$^{2}$, D-Trp$^{3}$, D-Arg$^{6}$, D-Ala$^{10}$]LHRH;

[N-Ac-D-pCl-Phe$^{1}$, D-pCl-Phe$^{2}$, D-Trp$^{3}$, D-Arg$^{6}$, D-Ala$^{10}$]LHRH;

[N-Ac-D-Nal(2)$^{1}$, D-pF-Phe$^{2}$, D-Trp$^{3}$, D-Arg$^{6}$]LHRH;

[N-Ac-D-Nal(2)$^{1}$, D-pF-Phe$^{2}$, D-Trp$^{3}$, oCl-Phe$^{5}$, D-Arg$^{6}$]LHRH;

[N-Ac-D-pBr-Phe$^{1}$, D-pCl-Phe$^{2}$, D-Trp$^{3}$, D-Arg$^{6}$, D-Ala$^{10}$]LHRH;

[N-Ac-D-Nal(2)$^{1}$, D-pCl-Phe$^{2}$, D-Trp$^{3}$, D-Arg$^{6}$, D-Ala$^{10}$]LHRH;

[N-Ac-D-Nal(2)$^{1}$, D-pCl-Phe$^{2}$, D-Pal(3)$^{3}$, D-Arg$^{6}$, D-Ala$^{10}$]LHRH;

[N-Ac-D-Nal(2)$^{1}$, D-pCl-Phe$^{2}$, D-Pal(3)$^{3}$, D-Arg$^{6}$, D-Trp$^{7}$, D-Ala$^{10}$]LHRH;

[N-Ac-D-Nal(2)$^{1}$, D-pCl-Phe$^{2}$, D-Pal(3)$^{3}$, D-Arg$^{6}$, D-Ile$^{7}$, D-Ala$^{10}$]LHRH;

[N-Ac-D-Nal(2)$^{1}$, D-pCl-Phe$^{2}$, D-Pal(3)$^{3}$, L-Arg$^{5}$, D-Glu(p-MeO-Phe)$^{6}$, D-Ala$^{10}$]LHRH;

[N-Ac-D-Nal(2)$^{1}$, D-α-Me-pCl-Phe$^{2}$, D-Pal(3)$^{3}$, iso-propyl-Lys$^{5,8}$, D-Tyr$^{6}$, D-Ala$^{10}$]LHRH;

[N-Ac-D-Nal(2)$^{1}$, D-αMe-pCl-Phe$^{2}$, D-Trp$^{3}$, Arg$^{5}$, D-Tyr$^{6}$, D-Ala$^{10}$]LHRH; and

[N-Ac-D-Nal(2)$^{1}$, D-αMe-pCl-Phe$^{2}$, D-Pal(3)$^{3}$, D-Arg$^{6}$, N-iPrLys$^{8}$, D-Ala$^{10}$]LHRH.

5. The use of any one of claims 1-4 wherein the medicament terminates pregnancy.

6. The use of any one of claims 1-4 wherein the medicament terminates heat.

7. The use of any one of claims 1-4 wherein the medicament is for a dog needing treatment of pyometritis and endometritis.